# FASCICULE DE BREVET EUROPEEN

(11) **EP 4 444 392 B1**
(45) Date de publication et mention de la délivrance du brevet: **12.11.2025**
(21) Numéro de dépôt: 22843853.7
(22) Date de dépôt: 07.12.2022
(51) Int. Cl.: A61M 11/02, A61M 15/00, A61M 15/08, A61M 11/00

(54) **DISPOSITIF DE DISTRIBUTION NASALE DE PRODUIT FLUIDE**
VORRICHTUNG ZUR NASALEN ABGABE EINES FLÜSSIGEN PRODUKTS
DEVICE FOR NASAL DELIVERY OF FLUID PRODUCT

(30) Priorité: 10.12.2021 FR 2113279
(43) Date de publication de la demande: 16.10.2024
(73) Titulaire: APTAR France SAS, 27110 Le Neubourg (FR)
(72) Inventeur: BIZET, Bruno, 71000 SANCÉ (FR); CHAMARANDE, Vincent, 71000 MÂCON (FR); GOUJON BROYER, Aurélie, 01570 MANZIAT (FR)
(74) Mandataire: CAPRI
(86) Numéro de dépôt international: PCT/FR2022/052269
(87) Numéro de publication internationale: WO 2023/105161

(56) Documents cités:
- WO-A1-01/85245
- WO-A1-03/035155
- WO-A1-2020/154182
- WO-A1-2021/069972
- CA-A1- 2 492 255
- FR-A1- 3 068 625
- GB-A- 2 536 259
- US-A1- 2008 295 834

## Description

La présente invention concerne un dispositif de distribution nasale de produit fluide, et plus particulièrement un dispositif pour distribuer dans une narine une dose de produit fluide pharmaceutique, notamment de liquide, contenue dans un réservoir, à l'aide d'un écoulement de gaz comprimé.

Les documents WO9946055 et WO0245866 divulguent des dispositifs de distribution nasale dans lesquels le produit fluide contenu dans le réservoir est entrainé par un écoulement d'air pressurisé créé par une chasse d'air actionnée manuellement par l'utilisateur. Le document WO2020154182 propose de remplacer la chasse d'air manuelle par un réservoir de gaz propulseur comprimé qui est ouvert lors de l'actionnement pour distribuer la dose de produit fluide. Le dispositif comporte une aiguille à double pointe, et lorsque l'utilisateur appuie sur le fond du réservoir, l'aiguille va percer d'une part le réservoir de produit fluide et d'autre part le réservoir de gaz comprimé, permettant ainsi au gaz comprimé d'entrainer et de distribuer la dose de produit fluide.

Le document GB2536259 décrit un autre dispositif de l'état de la technique, comportant un réservoir contenant un gaz propulseur comprimé.

Ces dispositifs connus présentent des inconvénients. En particulier, la précision de dosage des dispositifs qui mélangent de l'air ou du gaz au produit fluide n'est pas optimale, car l'expulsion de la dose complète n'est pas garantie avec ce type de dispositifs. De plus, l'utilisation d'un gaz propulseur comprimé est coûteux et potentiellement nocif pour l'environnement. De plus, de tels dispositifs, comportant généralement du métal et du plastique, sont plus complexes à recycler. WO2021/069972A1 divulgue les caractéristiques du préambule de la revendication 1.

La présente invention a pour but de fournir un dispositif de distribution nasale de produit fluide qui ne reproduit pas les inconvénients susmentionnés.

La présente invention a notamment pour but de fournir un tel dispositif qui garantisse l'expulsion de la totalité de la dose de produit fluide.

La présente invention a également pour but de fournir un tel dispositif qui utilise un écoulement de gaz pressurisé pour distribuer le produit fluide, sans que le gaz pressurisé ne contacte ledit produit fluide.

La présente invention a aussi pour but de fournir un tel dispositif qui favorise la déposition du produit fluide dans les parties éloignées de la narine, notamment les éthmoïdes.

La présente invention a également pour but de fournir un tel dispositif qui est dépourvu de gaz propulseur comprimé potentiellement nocif pour l'environnement.

La présente invention a aussi pour but de fournir un tel dispositif qui soit simple et peu coûteux à fabriquer, à assembler, à remplir et/ou à utiliser.

La présente a donc pour objet un dispositif de distribution de produit fluide comportant une tête de distribution dont l'extrémité axiale comporte un orifice de distribution, un réservoir contenant une dose de produit fluide, un système de génération d'un écoulement de gaz comprimé adapté à générer un écoulement de gaz comprimé lors de l'actionnement, et une partie intermédiaire reliant ledit réservoir audit système de génération d'un écoulement de gaz comprimé et comportant des moyens d'actionnement pour actionner ledit dispositif, ledit réservoir comprenant une partie mobile ou déformable adaptée pour expulser le produit fluide hors dudit réservoir, ladite partie mobile ou déformable étant actionnée par ledit écoulement de gaz comprimé généré lors de l'actionnement, de telle sorte que ledit écoulement de gaz comprimé n'entre pas en contact avec ledit produit fluide, ledit système de génération d'un écoulement de gaz comprimé étant formé par une chasse d'air comportant une chambre d'air dans laquelle coulisse un piston d'air, des moyens de chargement étant prévus pour déplacer ledit piston d'air dans ladite chambre d'air pour comprimer l'air contenu dans ladite chambre d'air.

Avantageusement, ladite chambre d'air comporte une ouverture obturée par un obturateur, ledit obturateur étant adapté à se déplacer et/ou à se déformer lors de l'actionnement, pour ouvrir ladite ouverture, générant ainsi un écoulement d'air comprimé.

Avantageusement, lesdits moyens de chargement sont formés par un capot monté pivotant sur ledit dispositif de telle sorte que lorsque ledit capot est déplacé d'une position fermée vers une position ouverte, ledit piston d'air est déplacé pour comprimer l'air contenu dans ladite chambre d'air.

Selon une première variante de réalisation avantageuse, ledit réservoir comporte un corps creux cylindrique contenant le produit fluide et pourvu d'une ouverture supérieure et d'une ouverture inférieure, ladite ouverture supérieure étant reliée audit orifice de distribution, et ladite ouverture inférieure étant fermée par un piston monté coulissant dans ledit corps creux cylindrique, ledit piston formant ladite partie mobile ou déformable du réservoir, de sorte que le produit fluide est distribué à travers ledit orifice de distribution lorsque ledit piston coulisse dans ledit corps creux cylindrique lors de l'actionnement.

Selon une seconde variante de réalisation avantageuse, ledit réservoir comporte une membrane ou poche déformable pourvue d'une ouverture supérieure reliée audit orifice de distribution, ladite membrane ou poche déformable formant ladite partie mobile ou déformable du réservoir, de sorte que le produit fluide est distribué à travers ledit orifice de distribution lorsque ladite membrane ou poche déformable est déformée lors de l'actionnement.

Selon l'invention, lesdits moyens d'actionnement comportent un bouton d'actionnement latéral, déplaçable radialement par rapport à ladite tête de distribution entre une position de repos et une position d'actionnement.

Avantageusement, lesdits moyens d'actionnement comportent un élément d'actionnement axial, déplaçable axialement par rapport à ladite tête de distribution entre une position de repos et une position d'actionnement.

Avantageusement, ladite partie intermédiaire comporte des moyens d'ouverture permettant, lors de l'actionnement, de libérer l'écoulement de gaz comprimé.

Selon une première variante de réalisation avantageuse, lesdits moyens d'ouverture comportent une plaque trouée, déplaçable latéralement ensemble avec un bouton d'actionnement latéral, ladite plaque obturant avant actionnement une sortie dudit système de génération d'un écoulement de gaz comprimé, et après actionnement, un trou de ladite plaque trouée vient se positionner face à ladite ouverture, laissant sortir l'écoulement de gaz comprimé.

Selon une seconde variante de réalisation avantageuse, lesdits moyens d'ouverture comportent un élément de poussée adapté à pousser une bille obturant avant actionnement une sortie dudit système de génération d'un écoulement de gaz comprimé.

Selon une troisième variante de réalisation avantageuse, lesdits moyens d'ouverture comportent un élément de perçage adapté à percer une membrane obturant avant actionnement une sortie dudit système de génération d'un écoulement de gaz comprimé.

Avantageusement, ledit élément de perçage est fixe par rapport audit réservoir.

En variante, ledit élément de perçage est mobile par rapport audit réservoir.

Avantageusement, ledit produit fluide est un médicament sous forme liquide.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique en section d'un dispositif de distribution de produit fluide selon un premier mode de réalisation avantageux, avant utilisation,
la figure 2 est une vue similaire à celle de la figure 1, après chargement de la chasse d'air et avant actionnement,
la figure 3 est une vue schématique en section d'un dispositif de distribution de produit fluide selon un second mode de réalisation avantageux, avant actionnement,
la figure 4 est une vue similaire à celle de la figure 3, après actionnement,
la figure 5 est une vue schématique en section d'un dispositif de distribution de produit fluide selon un troisième mode de réalisation avantageux, avant actionnement,
la figure 6 est une vue similaire à celle de la figure 5, après actionnement,
la figure 7 est une vue schématique en section d'un dispositif de distribution de produit fluide selon un quatrième mode de réalisation avantageux, avant actionnement, et
la figure 8 est une vue similaire à celle de la figure 7, après actionnement.

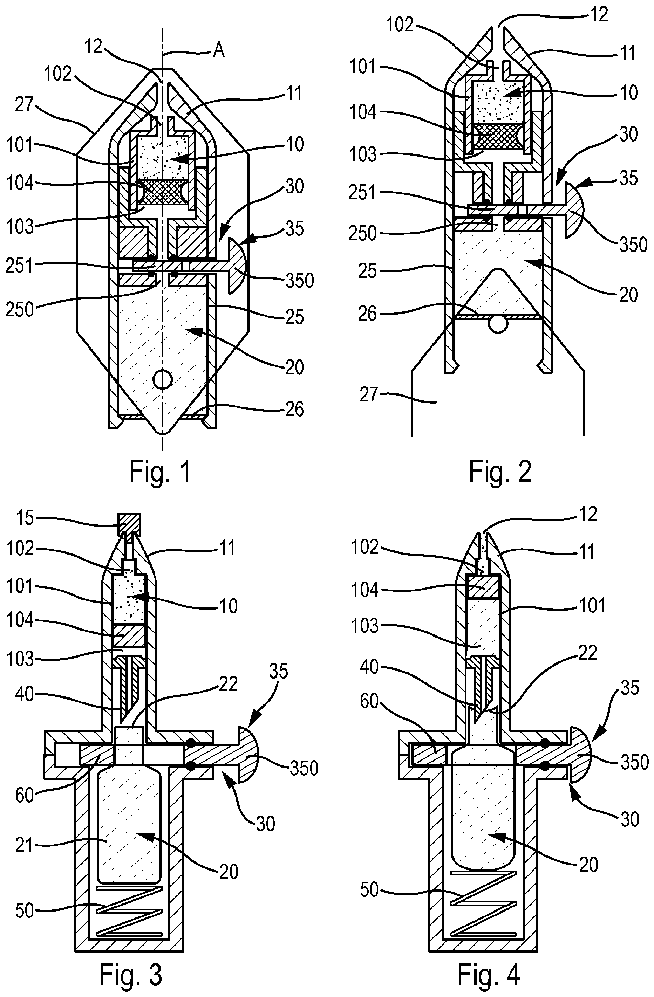

Il est entendu que dans la description ci-après, les termes "supérieur" et "inférieur" se réfèrent à la position droite du dispositif représenté sur les figures. Les termes "axial" et "radial" se réfèrent à l'axe central longitudinal A du dispositif, représenté sur la figure 1.

Le dispositif comporte trois parties principales, à savoir un réservoir 10 contenant une dose de produit fluide à distribuer, un système de génération d'un écoulement de gaz comprimé 20 adapté à générer un écoulement de gaz comprimé lors de l'actionnement pour expulser la dose de produit fluide lors de l'actionnement, et une partie intermédiaire 30 reliant le réservoir 10 au système de génération d'un écoulement de gaz comprimé 20 et permettant l'actionnement du dispositif.

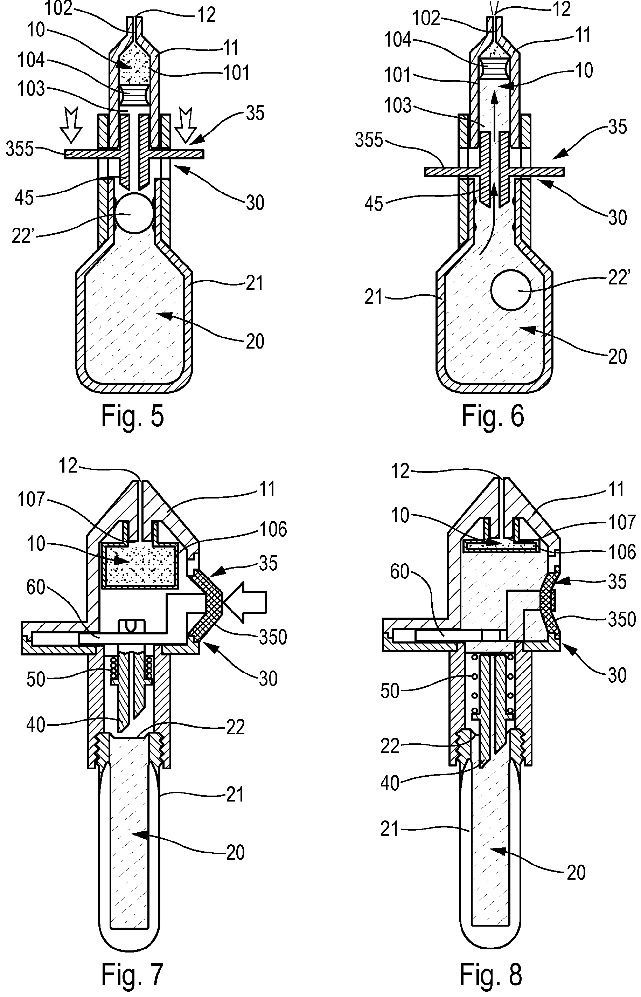

Le réservoir 10 est solidaire d'une tête de distribution 11 de forme axialement allongée, dont l'extrémité axiale comporte un orifice de distribution 12, et qui est destinée à être insérée dans la narine d'un utilisateur. Le réservoir 10 peut être formé directement dans la tête de distribution 11, comme représenté dans l'exemple des figures 5 et 6. En variante, comme visible sur les figures 1 à 4 et 7, 8, le réservoir 10 peut être formé séparément de la tête de distribution 11, en étant inséré et/ou fixé dans celle-ci.

Eventuellement, un profil de pulvérisation peut être prévu au niveau de l'orifice de distribution 12 pour réaliser une distribution du produit fluide sous forme de spray. Néanmoins, pour atteindre les parties plus lointaines du nez, telles que les éthmoïdes, il est généralement souhaitable de limiter la dispersion latérale du produit fluide, et un spray très large ne serait dans ce cas pas idéal. Une distribution sous forme de jet peut alors être avantageuse.

L'orifice de distribution 12 peut être fermé par un bouchon amovible 15 qui est retiré juste avant l'utilisation du dispositif, comme visible sur la figure 3. Dans l'exemple de la figure 1, c'est un capot 27 qui ferme et protège l'orifice de distribution 12 avant utilisation.

Le produit fluide est de préférence un médicament sous forme liquide, mais des produits fluides plus ou moins visqueux pourraient aussi être utilisés.

Selon l'invention, le réservoir 10 comprend une partie mobile ou déformable adaptée pour expulser le produit fluide hors du réservoir, ladite partie mobile ou déformable étant actionnée par ledit écoulement de gaz comprimé généré lors de l'actionnement, sans que ledit écoulement de gaz ne contacte le produit fluide.

Dans les modes de réalisation avantageux, visibles sur les figures 1 à 6, le réservoir 10 comporte un corps creux cylindrique 101 contenant le produit fluide et pourvu d'une ouverture supérieure 102 et d'une ouverture inférieure 103. L'ouverture supérieure 102 est reliée à l'orifice de distribution 12, et l'ouverture inférieure 103 est fermée par un piston 104 monté coulissant dans le corps creux cylindrique 101.

Dans ces modes de réalisation, le piston 104 forme la partie mobile ou déformable du réservoir. Le produit fluide, disposé dans le corps creux cylindrique 101 au-dessus du piston 104 est ainsi distribué à travers ladite ouverture supérieure 102 vers ledit orifice de distribution 12 lorsque le piston 104 coulisse dans le corps creux cylindrique 101 lors de l'actionnement.

Dans un autre mode de réalisation, visible sur les figures 7 et 8, le réservoir 10 comporte une membrane ou poche déformable 106. Le réservoir 10 comporte une ouverture supérieure 107 reliée à l'orifice de distribution 12.

Lors de l'actionnement, la membrane ou poche déformable 106 est déformée pour expulser le produit fluide à travers ladite ouverture supérieure 107 vers ledit orifice de distribution 12.

Dans ce mode de réalisation, la membrane ou poche déformable 106 forme la partie mobile ou déformable du réservoir.

Selon l'invention, le système de génération d'un écoulement de gaz comprimé 20 comporte une chasse d'air adaptée à générer un écoulement d'air comprimé.

Cette chasse d'air comporte une chambre d'air 25 dans laquelle coulisse un piston d'air 26. La chambre d'air 25 comporte une ouverture 250 obturée par un obturateur 251. Des moyens de chargement 27 sont prévus pour déplacer le piston d'air 26 dans la chambre d'air 25 pour ainsi comprimer l'air contenu dans ladite chambre d'air 25.

Dans l'exemple des figures 1 et 2, ces moyens de chargement sont formés par le capot monté pivotant de telle sorte que lorsque le capot 27 est déplacé de sa position fermée, visible sur la figure 1, vers sa position ouverte, visible sur la figure 2, le piston d'air 26 est déplacé pour comprime l'air contenu dans la chambre d'air 25.

Lors de l'actionnement, l'obturateur 251 est déplacé et/ou déformé pour ouvrir l'ouverture 250, générant ainsi un écoulement d'air comprimé.

Dans les autres modes de réalisation des figures 3 à 8, le système de génération d'un écoulement de gaz comprimé 20 comporte un réservoir de gaz comprimé 21 contenant du gaz comprimé. Il est à noter que cette solution avec du gaz propulseur comprimé n'est pas couverte par la présente invention, mais ces modes de réalisation des figures 3 à 8 sont intéressants pour d'autres aspects du dispositif, et peuvent également s'appliquer à un dispositif utilisant une chasse d'air, c'est-à-dire dépourvus de gaz propulseur comprimé potentiellement néfaste pour l'environnement.

Ce réservoir de gaz comprimé 21 est obturé par des moyens de fermeture 22, 22', par exemple une membrane 22 dans les exemples des figures 3, 4 et 7, 8, ou une bille 22' dans l'exemple des figures 5, 6.

Lors de l'actionnement, les moyens de fermeture 22, 22' sont ouverts, par exemple la membrane 22 est percée ou la bille 22' est expulsée de sa position d'obturation, ce qui libère le gaz comprimé contenu dans le réservoir de gaz comprimé 21, générant ainsi un écoulement de gaz comprimé.

La partie intermédiaire 30 comporte des moyens d'actionnement 35 pour actionner le dispositif.

Dans les exemples des figures 1 à 4, 7 et 8, ces moyens d'actionnement 35 comportent un bouton d'actionnement latéral 350, déplaçable radialement par rapport à la tête de distribution 11 entre une position de repos et une position d'actionnement.

Dans l'exemple des figures 5 et 6, les moyens d'actionnement 35 comportent un élément d'actionnement axial 355, déplaçable axialement par rapport à la tête de distribution 11 entre une position de repos et une position d'actionnement.

La partie intermédiaire 30 comporte aussi des moyens d'ouverture permettant lors de l'actionnement de déplacer l'obturateur 251 ou d'ouvrir les moyens de fermeture 22, 22', pour libérer l'écoulement de gaz comprimé.

Dans l'exemple des figures 1 et 2, ces moyens d'ouverture comportent une plaque trouée 251, déplaçable latéralement ensemble avec le bouton d'actionnement latéral 350. Avant actionnement, la plaque 251 obture la chambre d'air 25, et après actionnement, le trou de la plaque trouée 251 vient se positionner face à l'ouverture 250 de la chambre d'air 25, laissant sortir l'air comprimé.

Dans les exemples des figures 3, 4 et 7, 8, un élément de perçage 40 vient percer la membrane de fermeture 22 du réservoir de gaz comprimé 21. Avant actionnement, la membrane 22 du réservoir de gaz comprimé 21 est maintenue en éloignement de l'élément de perçage 40 par un organe de blocage 60 solidaire du bouton d'actionnement latéral 350.

Dans l'exemple des figures 3 et 4, l'élément de perçage 40 est fixe et le réservoir de gaz comprimé 21 est sollicité par un ressort 50 contre ledit élément de perçage 40. L'organe de blocage 60 coopère avec le réservoir de gaz comprimé 21 pour empêcher son déplacement, jusqu'au moment de l'actionnement, qui libère l'organe de blocage 60.

Dans l'exemple des figures 7 et 8, le réservoir de gaz comprimé 21 est fixe et l'élément de perçage 40 est sollicité par un ressort 50 contre ledit réservoir de gaz comprimé 21. L'organe de blocage 60 coopère avec l'élément de perçage 40 pour empêcher son déplacement, jusqu'au moment de l'actionnement, qui libère l'organe de blocage 60.

Dans l'exemple des figures 5 et 6, un élément de poussée 45 vient pousser la bille 22' du réservoir de gaz comprimé 21. Avant actionnement, l'élément de poussée 45 est maintenu en éloignement de la bille 22', par exemple par un ressort.

Lorsque l'utilisateur souhaite utiliser le dispositif, il place la tête de distribution 11 dans une narine, et appuie sur les moyens d'actionnement 35 pour déclencher les moyens d'ouverture qui vont actionner le système de génération d'un écoulement de gaz comprimé 20, générant ainsi un écoulement de gaz comprimé.

Dans les exemples des figures 1 à 4, 7 et 8, l'écoulement de gaz comprimé va déplacer le piston 104 dans le réservoir 10 pour expulser la dose de produit fluide.

Dans l'exemples des figures 5 et 6, l'écoulement de gaz comprimé va déformer la membrane ou poche déformable du réservoir 10 pour expulser la dose de produit fluide.

Ainsi, l'écoulement de gaz comprimé ne se mélange pas au produit fluide, ce qui favorise une meilleure précision de dosage.

Le dispositif selon l'invention améliore la distribution du produit fluide dans la narine, en permettant au produit d'atteindre des parties plus lointaines, telles que les éthmoïdes.

Le dispositif selon l'invention est notamment adapté pour être utilisé dans le traitement des rhinites allergiques et du syndrome de sensibilisation centrale.

Dans le cadre d'une rhinite allergique, le produit fluide peut être choisi dans la liste suivante, donnée à titre d'exemple non limitatif : fluticasone, mometasone, fluticasone furoate, budésonide, béclometasone, azelastine fluticasone, azelastine, tixocortol, triamcinolone acetonide, ipratropium bromide, prednisolone, ciclesonide, flunisolide, levocabastine, azelastine fluticasone furoate, olopatadine, azelastine mometasone, mometasone olopatadine, fluticasone olopatadine.

Dans le cadre d'un syndrome de sensibilisation centrale, le produit fluide peut être choisi dans la liste suivante, donnée à titre d'exemple non limitatif : sumatriptan, zolmitriptan, naloxone, fentanyl, esketamine, midazolam, butorphanol, dihydroergotamine, diazépam, tapentadol, alfentanil, diamorphine, ketorolac.

La présente invention a été décrite en référence à divers modes et variantes de réalisation, mais il est entendu qu'un homme du métier peut y apporter toutes modifications, sans sortir du cadre de la présente invention telle que défini par les revendications annexées.

En particulier, des caractéristiques décrites en référence à un mode de réalisation pourraient s'appliquer également à d'autres modes de réalisation. Ainsi, par exemple, et de manière non limitative, le mode de réalisation des figures 5 et 6 pourrait comporter une membrane 22 à la place d'une bille 22'. De même, la poche déformable du mode de réalisation des figures 7 et 8 pourrait être remplacée par un réservoir avec piston, tel que décrit dans les modes de réalisation des figures 1 à 6.

Bien entendu, d'autres variantes de réalisation sont également envisageables, sans sortir du cadre de la présente invention, tel que défini par les revendications annexées.

## Revendications

1. Dispositif de distribution de produit fluide comportant une tête de distribution (11) dont l'extrémité axiale comporte un orifice de distribution (12), un réservoir (10) contenant une dose de produit fluide, un système de génération d'un écoulement de gaz comprimé (20) adapté à générer un écoulement de gaz comprimé lors de l'actionnement, et une partie intermédiaire (30) reliant ledit réservoir (10) audit système de génération d'un écoulement de gaz comprimé (20) et comportant des moyens d'actionnement (35) pour actionner ledit dispositif, ledit réservoir (10) comprenant une partie mobile ou déformable (104, 106) adaptée pour expulser le produit fluide hors dudit réservoir (10), ladite partie mobile ou déformable (104, 106) étant actionnée par ledit écoulement de gaz comprimé généré lors de l'actionnement, de telle sorte que ledit écoulement de gaz comprimé n'entre pas en contact avec ledit produit fluide, ledit système de génération d'un écoulement de gaz comprimé (20) étant formé par une chasse d'air comportant une chambre d'air (25) dans laquelle coulisse un piston d'air (26), des moyens de chargement (27) étant prévus pour déplacer ledit piston d'air (26) dans ladite chambre d'air (25) pour comprimer l'air contenu dans ladite chambre d'air (25), **caractérisé en ce que** lesdits moyens d'actionnement (35) comportent un bouton d'actionnement latéral (350), déplaçable radialement par rapport à ladite tête de distribution (11) entre une position de repos et une position d'actionnement.

2. Dispositif selon la revendication 1, dans lequel ladite chambre d'air (25) comporte une ouverture (250) obturée par un obturateur (251), ledit obturateur (251) étant adapté à se déplacer et/ou à se déformer lors de l'actionnement, pour ouvrir ladite ouverture (250), générant ainsi un écoulement d'air comprimé.

3. Dispositif selon la revendication 1 ou 2, dans lequel lesdits moyens de chargement sont formés par un capot monté pivotant sur ledit dispositif de telle sorte que lorsque ledit capot (27) est déplacé d'une position fermée vers une position ouverte, ledit piston d'air (26) est déplacé pour comprimer l'air contenu dans ladite chambre d'air (25).

4. Dispositif selon l'une quelconque des revendications précédentes, dans lequel ledit réservoir (10) comporte un corps creux cylindrique (101) contenant le produit fluide et pourvu d'une ouverture supérieure (102) et d'une ouverture inférieure (103), ladite ouverture supérieure (102) étant reliée audit orifice de distribution (12), et ladite ouverture inférieure (103) étant fermée par un piston (104) monté coulissant dans ledit corps creux cylindrique (101), ledit piston (104) formant ladite partie mobile ou déformable du réservoir, de sorte que le produit fluide est distribué à travers ledit orifice de distribution (12) lorsque ledit piston (104) coulisse dans ledit corps creux cylindrique (101) lors de l'actionnement.

5. Dispositif selon l'une quelconque des revendications 1 à 3, dans lequel ledit réservoir (10) comporte une membrane ou poche déformable (106) pourvue d'une ouverture supérieure (107) reliée audit orifice de distribution (12), ladite membrane ou poche déformable (106) formant ladite partie mobile ou déformable du réservoir, de sorte que le produit fluide est distribué à travers ledit orifice de distribution (12) lorsque ladite membrane ou poche déformable (106) est déformée lors de l'actionnement.

6. Dispositif selon l'une quelconque des revendications précédentes, dans lequel ladite partie intermédiaire (30) comporte des moyens d'ouverture permettant, lors de l'actionnement, de libérer l'écoulement de gaz comprimé.

7. Dispositif selon la revendication 6, dans lequel lesdits moyens d'ouverture comportent une plaque trouée (251), déplaçable latéralement ensemble avec un bouton d'actionnement latéral (350), ladite plaque (251) obturant avant actionnement une sortie (250) dudit système de génération d'un écoulement de gaz comprimé (20), et après actionnement, un trou de ladite plaque trouée (251) vient se positionner face à ladite ouverture (250), laissant sortir l'écoulement de gaz comprimé.

8. Dispositif selon la revendication 6, dans lequel lesdits moyens d'ouverture comportent un élément de poussée (45) adapté à pousser une bille (22') obturant avant actionnement une sortie dudit système de génération d'un écoulement de gaz comprimé (20).

9. Dispositif selon la revendication 6, dans lequel lesdits moyens d'ouverture comportent un élément de perçage (40) adapté à percer une membrane (22) obturant avant actionnement une sortie dudit système de génération d'un écoulement de gaz comprimé (20).

10. Dispositif selon la revendication 9, dans lequel ledit élément de perçage (40) est fixe par rapport audit réservoir (10).

11. Dispositif selon la revendication 9, dans lequel ledit élément de perçage (40) est mobile par rapport audit réservoir (10).

12. Dispositif selon l'une quelconque des revendications précédentes, dans lequel ledit produit fluide est un médicament sous forme liquide.

## Patentansprüche

1. Vorrichtung zur Abgabe eines fluiden Produkts, aufweisend einen Abgabekopf (11), dessen axiales Ende eine Abgabeöffnung (12) aufweist, einen Behälter (10), der eine Dosis eines fluiden Produkts enthält, ein System zur Erzeugung eines Druckgasstroms (20), der dazu geeignet ist, bei Betätigung einen Druckgasstrom zu erzeugen, und einen Zwischenabschnitt (30), der den Behälter (10) mit dem System zur Erzeugung eines Druckgasstroms verbindet (20) und Betätigungsmittel (35) zum Betätigen der Vorrichtung aufweist, wobei der Behälter (10) einen beweglichen oder verformbaren Teil (104, 106) umfasst, der dazu geeignet ist, das fluide Produkt aus dem Behälter (10) auszustoßen, wobei der bewegliche oder verformbare Teil (104, 106) durch den bei der Betätigung erzeugten Druckgasstrom betätigt wird, so dass der Druckgasstrom nicht mit dem fluiden Produkt in Kontakt kommt, wobei das System zur Erzeugung eines Druckgasstroms (20) von einem Luftaustritt gebildet wird, der eine Luftkammer (25) aufweist, in der ein Luftkolben (26) gleitet, wobei Lademittel (27) vorgesehen sind, um den Luftkolben (26) in der Luftkammer (25) zu bewegen, um die in der Luftkammer (25) enthaltene Luft zu verdichten, **dadurch gekennzeichnet, dass** die Betätigungsmittel (35) einen seitlichen Betätigungsknopf (350) aufweisen, der radial in Bezug auf den Abgabekopf (11) zwischen einer Ruhestellung und einer Betätigungsstellung bewegbar ist.

2. Vorrichtung nach Anspruch 1, wobei die Luftkammer (25) eine Öffnung (250) aufweist, die von einem Verschluss (251) verschlossen wird, wobei der Verschluss (251) geeignet ist, sich bei Betätigung zu bewegen und/oder zu verformen, um die Öffnung (250) zu öffnen, wodurch ein Druckluftstrom erzeugt wird.

3. Vorrichtung nach Anspruch 1 oder 2, wobei die Lademittel von einer schwenkbar an der Vorrichtung angebrachten Abdeckung gebildet werden, so dass, wenn die Abdeckung (27) aus einer geschlossenen Position in eine geöffnete Position bewegt wird, der Luftkolben (26) bewegt wird, um die in der Luftkammer (25) enthaltene Luft zu verdichten.

4. Vorrichtung nach einem der vorstehenden Ansprüche, wobei der Behälter (10) einen zylindrischen Hohlkörper (101) aufweist, der das fluide Produkt enthält und mit einer oberen Öffnung (102) und einer unteren Öffnung (103) versehen ist, wobei die obere Öffnung (102) mit der Abgabeöffnung (12) verbunden ist und die untere Öffnung (103) durch einen Kolben (104) verschlossen ist, der in dem zylindrischen Hohlkörper (101) gleitend angebracht ist, wobei der Kolben (104) den beweglichen oder verformbaren Teil des Behälters bildet, so dass das fluide Produkt durch die Abgabeöffnung (12) abgegeben wird, wenn der Kolben (104) bei Betätigung in dem zylindrischen Hohlkörper (101) gleitet.

5. Vorrichtung nach einem der Ansprüche 1 bis 3, wobei der Behälter (10) eine verformbare Membran oder einen verformbaren Beutel (106) aufweist, der mit einer oberen Öffnung (107) versehen ist, die mit der Abgabeöffnung (12) verbunden ist, wobei die verformbare Membran oder der verformbare Beutel (106) den beweglichen oder verformbaren Teil des Behälters bildet, so dass das fluide Produkt durch die Abgabeöffnung (12) abgegeben wird, wenn die verformbare Membran oder der verformbare Beutel (106) bei Betätigung verformt wird.

6. Vorrichtung nach einem der vorstehenden Ansprüche, wobei der Zwischenabschnitt (30) Öffnungsmittel umfasst, die bei Betätigung den Druckgasstrom freigeben.

7. Vorrichtung nach Anspruch 6, wobei die Öffnungsvorrichtung eine Lochplatte (251) umfasst, die zusammen mit einem seitlichen Betätigungsknopf (350) seitlich bewegbar ist, wobei die Platte (251) vor der Betätigung einen Auslass (250) des Systems zur Erzeugung eines Druckgasstroms (20) verschließt und nach der Betätigung ein Loch der Lochplatte (251) gegenüber der Öffnung (250) positioniert wird, wodurch der Druckgasstrom austreten kann.

8. Vorrichtung nach Anspruch 6, wobei die Öffnungsmittel ein Druckelement (45) umfassen, das geeignet ist, auf eine Kugel (22') zu drücken, die vor der Betätigung einen Auslass des Systems zur Erzeugung eines Druckgasstroms (20) verschließt.

9. Vorrichtung nach Anspruch 6, wobei die Öffnungsmittel ein Durchstechelement (40) umfassen, das geeignet ist, eine Membran (22) zu durchstechen, die vor Betätigung einen Auslass des Systems zur Erzeugung eines Druckgasstroms (20) verschließt.

10. Vorrichtung nach Anspruch 9, wobei das Durchstechelement (40) in Bezug auf den Behälter (10) feststehend ist.

11. Vorrichtung nach Anspruch 9, wobei das Durchstechelement (40) in Bezug auf den Behälter (10) beweglich ist.

12. Vorrichtung nach einem der vorstehenden Ansprüche, wobei das fluide Produkt ein Arzneimittel in flüssiger Form ist.

## Claims

1. A device for dispensing fluid product, comprising a dispensing head (11) the axial end of which comprises a dispensing orifice (12), a reservoir (10) containing a dose of fluid product, a system for generating a flow of compressed gas (20) adapted to generate a flow of compressed gas during actuation, and an intermediate portion (30) connecting said reservoir (10) to said system for generating a flow of compressed gas (20) and comprising actuating means (35) for actuating said device, said reservoir (10) comprising a mobile or deformable portion (104, 106) adapted to expel the fluid product out of said reservoir (10), said mobile or deformable portion (104, 106) being actuated by said flow of compressed gas generated during the actuation, in a manner such that said flow of compressed gas does not come into contact with said fluid product, said system for generating a flow of compressed gas (20) being formed by an air blaster comprising an air chamber (25) in which an air piston (26) slides, loading means (27) being provided in order to displace said air piston (26) in said air chamber (25) in order to compress the air contained in said air chamber (25), **characterized in that** said actuating means (35) comprise a lateral actuating button (350) which is radially displaceable with respect to said dispensing head (11) between a rest position and an actuation position.

2. The device as claimed in claim 1, in which said air chamber (25) comprises an opening (250) shut off by a shutter (251), said shutter (251) being adapted to be displaced and/or to be deformed during the actuation in order to open said opening (250), thereby generating a flow of compressed air.

3. The device as claimed in claim 1 or claim 2, in which said loading means are formed by a cover which is pivotably mounted on said device in a manner such that when said cover (27) is displaced from a closed position towards an open position, said air piston (26) is displaced in order to compress the air contained in said air chamber (25).

4. The device as claimed in any one of the preceding claims, in which said reservoir (10) comprises a hollow cylindrical body (101) containing the fluid product and provided with an upper opening (102) and a lower opening (103), said upper opening (102) being connected to said dispensing orifice (12), and said lower opening (103) being closed by a piston (104) which is slidably mounted in said hollow cylindrical body (101), said piston (104) forming said mobile or deformable portion of the reservoir, in a manner such that the fluid product is dispensed through said dispensing orifice (12) when said piston (104) slides in said hollow cylindrical body (101) during the actuation.

5. The device as claimed in any one of claims 1 to 3, in which said reservoir (10) comprises a deformable membrane or pouch (106) provided with an upper opening (107) which is connected to said dispensing orifice (12), said deformable membrane or pouch (106) forming said mobile or deformable portion of the reservoir, in a manner such that the fluid product is dispensed through said dispensing orifice (12) when said deformable membrane or pouch (106) is deformed during the actuation.

6. The device as claimed in any one of the preceding claims, in which said intermediate portion (30) comprises opening means which, during the actuation, enable the compressed gas to be released.

7. The device as claimed in claim 6, in which said opening means comprise an apertured plate (251) which is laterally displaceable together with a lateral actuating button (350), said plate (251) shutting off an outlet (250) of said system for generating a flow of compressed gas (20) before actuation, and after actuation, an aperture of said apertured plate (251) becomes positioned facing said opening (250), letting out the flow of compressed gas.

8. The device as claimed in claim 6, in which said opening means comprise a push element (45) adapted to push a bead (22') shutting off an outlet of said system for generating a flow of compressed gas (20) before actuation.

9. The device as claimed in claim 6, in which said opening means comprise a piercing element (40) adapted to pierce a membrane (22) shutting off an outlet of said system for generating a flow of compressed gas (20) before actuation.

10. The device as claimed in claim 9, in which said piercing element (40) is fixed with respect to said reservoir (10).

11. The device as claimed in claim 9, in which said piercing element (40) is mobile with respect to said reservoir (10).

12. The device as claimed in any one of the preceding claims, in which said fluid product is a drug in liquid form.
